(19) Europäisches Patentamt
European Patent Office
Office européen des brevets

(11) **EP 4 517 314 A1**

(12) **EUROPEAN PATENT APPLICATION**
published in accordance with Art. 153(4) EPC

(43) Date of publication:
**05.03.2025 Bulletin 2025/10**

(21) Application number: **23796012.5**

(22) Date of filing: **03.04.2023**

(51) International Patent Classification (IPC):
**G01N 27/12** (2006.01)

(52) Cooperative Patent Classification (CPC):
**G01N 27/12**

(86) International application number:
**PCT/JP2023/013804**

(87) International publication number:
**WO 2023/210265 (02.11.2023 Gazette 2023/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.04.2022 JP 2022073680**

(71) Applicant: **Niterra Co., Ltd.
Nagoya-shi, Aichi 461-0005 (JP)**

(72) Inventors:
• **KONDO Tomonori
Nagoya-shi, Aichi 461-0005 (JP)**

• **TAKAKURA Masahiro
Nagoya-shi, Aichi 461-0005 (JP)**
• **SUDA Masanori
Nagoya-shi, Aichi 461-0005 (JP)**
• **HANAWA Yuichiro
Nagoya-shi, Aichi 461-0005 (JP)**
• **KURODA Ryo
Nagoya-shi, Aichi 461-0005 (JP)**

(74) Representative: **Diehl & Partner
Patent- und Rechtsanwaltskanzlei mbB
Erika-Mann-Straße 9
80636 München (DE)**

(54) **GAS SENSOR AND METHOD FOR PRODUCING GAS SENSOR**

(57)     A gas sensor having enhanced responsiveness is provided. A gas sensor (10) includes an electrode portion (50) which includes one or more electrode pairs (70) each having a first electrode (71) and a second electrode (72), the first electrode (71) and the second electrode (72) being opposed to each other in a first direction with a gap formed therebetween; and a sensitive membrane (60). When the product of an inter-electrode distance in the first direction between the first electrode (71) and the second electrode (72) and an electrode area of the first electrode (71) in a cut surface orthogonal to the first direction is defined as an inter-electrode volume, a division value obtained by dividing, by the inter-electrode volume, a voltage applied to the electrode portion (50) by a voltage application section (80) is greater than $4.2 \times 10^{16}$ V/m$^3$ and not greater than $2.0 \times 10^{25}$ V/m$^3$. The electrode area is equal to or greater than $2.5 \times 10^{-15}$ m$^2$.

*FIG. 1*

EP 4 517 314 A1

**Description**

TECHNICAL FIELD

The present disclosure relates to a gas sensor and a method for manufacturing a gas sensor.

BACKGROUND ART

In a conventional chemical sensor using a MEMS, a pair of electrodes is provided, and the distance between the electrodes is several micrometers even when the distance is the shortest. Although such a sensor exhibits sensitivity, its responsiveness is poor, and a long time is needed for concentration detection. Therefore, it was difficult to perform real-time measurement at intervals of several seconds to several tens of seconds. Meanwhile, as shown in Patent Literature 1, there has been reported a gas sensor which uses a nano-gap electrode having an inter-electrode distance of about several nm to 100 nm and which has improved responsiveness.

CITATION LIST

PATENT LITERATURE

Patent Literature 1: JP2021-32746A

SUMMARY OF INVENTION

TECHNICAL PROBLEM

Although the sensor disclosed in Patent Literature 1 exhibited quick response when exposed to a gas to be detected, when the sensor was actually used, a long time was needed to recover its sensor characteristics. Also, since the mechanism of enhancing its responsiveness has not yet been elucidated, knowledge about a sensor configuration for obtaining quick response has been insufficient.

The present disclosure provides a gas sensor having enhanced responsiveness.

SOLUTION TO PROBLEM

A gas sensor, which is a first aspect of the present disclosure, is characterized by comprising:

an electrode portion which includes one or more electrode pairs each having a first electrode and a second electrode, the first electrode and the second electrode being opposed to each other in a first direction in the electrode pair, with a gap formed therebetween;
a sensitive membrane disposed between the first electrode and the second electrode in the electrode pair; and
a voltage application section which applies a voltage to the electrode portion such that the voltage is applied between the first electrode and the second electrode,
wherein, when the product of an inter-electrode distance in the first direction between the first electrode and the second electrode and an electrode area of the first electrode in a cut surface orthogonal to the first direction is defined as an inter-electrode volume, a division value obtained by dividing, by the inter-electrode volume, the voltage applied to the electrode portion by the voltage application section is greater than $4.2 \times 10^{16}$ V/m$^3$ and not greater than $2.0 \times 10^{25}$ V/m$^3$ and
wherein the electrode area is equal to or greater than $2.5 \times 10^{-15}$ m$^2$.

A gas sensor manufacturing method, which is a second aspect of the present disclosure, is a method for manufacturing the above-described gas sensor, wherein the sensitive membrane is formed by means of physical vapor deposition or chemical vapor deposition.

In the gas sensor manufacturing method, which is the second aspect of the present disclosure, heat treatment is performed for the sensitive membrane after being formed.

ADVANTAGEOUS EFFECT OF INVENTION

The technique according to the present disclosure can enhance responsiveness.

BRIEF DESCRIPTION OF DRAWINGS

[FIG. 1] FIG. 1 is a side sectional view schematically showing a gas sensor of a first embodiment.
[FIG. 2] FIG. 2 is a partial enlarged view of the gas sensor of FIG. 1.
[FIG. 3] FIG. 3 is a plan view SEM image of electrode pairs of the gas sensor.
[FIG. 4] FIG. 4 is a plan view SEM image of a sensitive membrane of the gas sensor.
[FIG. 5] FIG. 5 is a schematic view used for describing an electrode pair and its inter-electrode volume.
[FIG. 6] FIG. 6 is an explanatory view used for describing a method for measuring the width and height of a first electrode in its cut surface.
[FIG. 7] FIG. 7 is a process chart used for describing a process of manufacturing the gas sensor.
[FIG. 8] FIG. 8 is a process chart which is continued from FIG. 7 and is used for describing the process of manufacturing the gas sensor.
[FIG. 9] FIG. 9 is an explanatory view used for briefly describing a sensor evaluation device.
[FIG. 10] FIG. 10(A) is an explanatory graph showing changes with time in the output of a gas sensor of Comparative Example 2, and FIG. 10(B) is an explanatory graph showing changes with time in the output of a gas sensor of Experimental Example 4.
[FIG. 11] FIG. 11(A) is an explanatory view used for describing depletion layers of tin oxide before exposure of the gas sensor to a gas to be detected, and FIG. 11(B) is an explanatory view used for describing depletion layers of tin oxide after exposure of the gas sensor to the gas to be detected.

DESCRIPTION OF EMBODIMENTS

In the below, embodiments of the present disclosure will be listed and illustrated.

[1] A gas sensor of the present disclosure comprises:

an electrode portion which includes one or more electrode pairs each having a first electrode and a second electrode, the first electrode and the second electrode being opposed to each other in a first direction in the electrode pair, with a gap formed therebetween;
a sensitive membrane disposed between the first electrode and the second electrode in the electrode pair; and
a voltage application section which applies a voltage to the electrode portion such that the voltage is applied between the first electrode and the second electrode,
wherein, when the product of an inter-electrode distance in the first direction between the first electrode and the second electrode and an electrode area of the first electrode in a cut surface orthogonal to the first direction is defined as an inter-electrode volume, a division value obtained by dividing, by the inter-electrode volume, the voltage applied to the electrode portion by the voltage application section is greater than $4.2 \times 10^{16}$ V/m$^3$ and not greater than $2.0 \times 10^{25}$ V/m$^3$ and
wherein the electrode area is equal to or greater than $2.5 \times 10^{-15}$ m$^2$.
In this configuration, since the division value obtained by dividing the voltage applied to the electrode portion by the inter-electrode volume is greater than $4.2 \times 10^{16}$ V/m$^3$ and not greater than $2.0 \times 10^{25}$ V/m$^3$, and the electrode area is equal to or greater than $2.5 \times 10^{-15}$ m$^2$, the response speed (rising and returning speeds) of the outputted electric signal can be increased.

[2] In the above-described gas sensor,

the electrode portion includes 4 or more electrode pairs, and
the electrode area is the total area of cross sections of all the first electrodes in a cut surface obtained by cutting all the first electrodes at a position which is offset in the first direction from their end portions on the second electrode side by an amount of 10 nm to 100 nm.
Since the gas sensor described in the above paragraph [2] has 4 or more electrode pairs and is configured such that the sum total of the electrode areas becomes equal to or greater than $2.5 \times 10^{-15}$ m$^2$, the sum total of the electrode areas becomes greater than that of the conventional technique. Thus, the response speed (rising and returning speeds) of the outputted electric signal can be increased.

[3] In the above-described gas sensor,

the division value is not less than $6.0\times10^{19}$ V/m$^3$ and not greater than $2.0\times10^{25}$ V/m$^3$.

In the gas sensor described in the above paragraph [3], since the division value is not less than $6.0\times10^{19}$ V/m$^3$ and not greater than $2.0\times10^{25}$ V/m$^3$, the response speed (rising and returning speeds) of the outputted electric signal can be further increased.

[4] In the above-described gas sensor,

the inter-electrode distance which is the smallest among all the electrode pairs in the electrode portion is 5 nm to 100 nm.

In the gas sensor described in the above paragraph [4], since a tunnel current effect is obtained, the response speed (rising and returning speeds) of the outputted electric signal can be further increased.

[5] In the above-described gas sensor,

the total of widths of the first electrodes of all the electrode pairs in the electrode portion is equal to or greater than 20 nm.

In the gas sensor described in the above paragraph [5], since the resistance between the first electrodes and the second electrodes can be reduced, the response speed (rising and returning speeds) of the outputted electric signal can be further increased.

[6] In the above-described gas sensor, the sensitive membrane is a dense body.

In the gas sensor described in the above paragraph [6], since the sensitive membrane is densified, movement of electrons in depletion layers becomes easy, whereby the response speed (rising and returning speeds) of the outputted electric signal can be increased.

[7] In the above-described gas sensor, at least some particles which constitute the sensitive membrane are electrically coupled with one another.

In the gas sensor described in the above paragraph [7], since the particles which constitute the sensitive membrane are coupled with one another so as to enable electrical conduction, the contact resistance between the particles can be reduced. Accordingly, movement of electrons in depletion layers becomes easy.

[8] A method for manufacturing the above-described gas sensor in which the sensitive membrane is formed by means of physical vapor deposition or chemical vapor deposition.

In the gas sensor described in the above paragraph [8], since a homogeneous sensitive membrane can be formed, stable output of the electric signal becomes possible.

[9] In the above-described gas sensor manufacturing method, heat treatment is performed for the sensitive membrane after being formed.

In the gas sensor described in the above paragraph [9], since the particles which constitute the sensitive membrane are easily coupled with one another, the contact resistance between the particles can be reduced, whereby movement of electrons in depletion layers becomes easy.

<First embodiment >

1-1. Structure of gas sensor

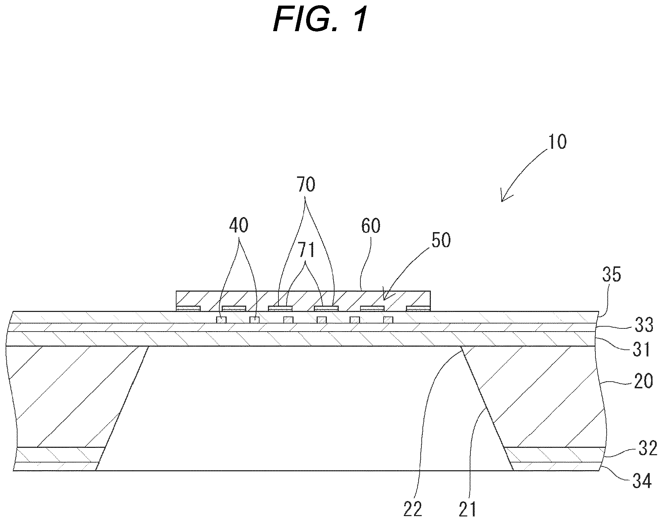

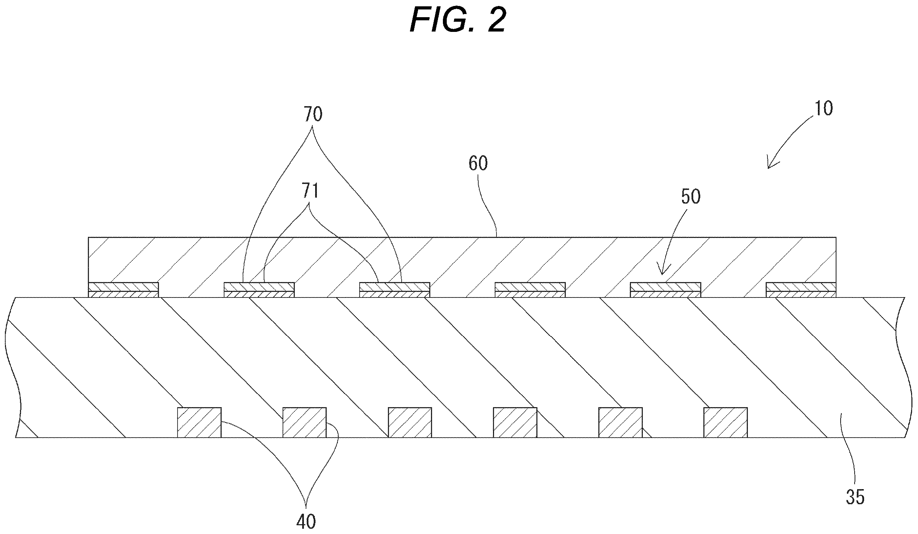

A first embodiment in which the present disclosure is embodied will now be described with reference to FIGS. 1 to 11. A gas sensor 10 of the first embodiment shown in FIG. 1 is one example of the gas sensor of the present disclosure. In the following description, for convenience of explanation, the upper and lower directions appearing in FIGS. 1 and 2 will be defined as the upper and lower directions. However, the upper and lower directions are not required to coincide with the upper and lower directions in a state in which the gas sensor 10 is actually disposed.

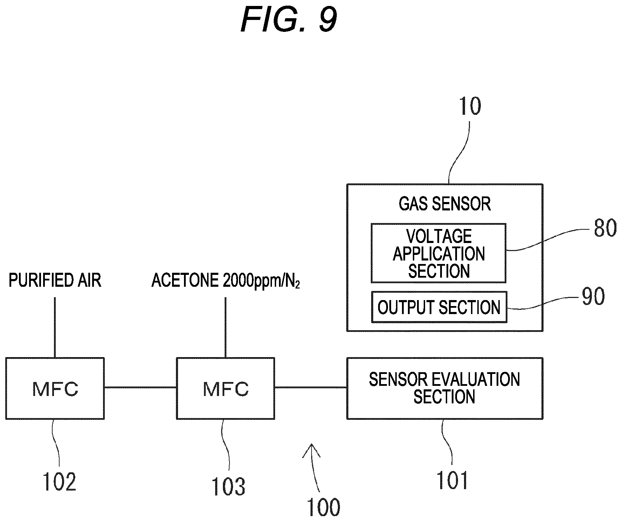

The gas sensor 10 includes a substrate 20, insulating layers 31 to 35, heating elements 40, an electrode portion 50, a sensitive membrane 60, a voltage application section 80 (see FIG. 9), and an output section 90 (see FIG. 9). The electrode portion 50 includes one or more electrode pairs 70 each having a first electrode 71 and a second electrode 72. The electrode portion 50 preferably includes four or more electrode pairs 70, more preferably 100 or more electrode pairs 70.

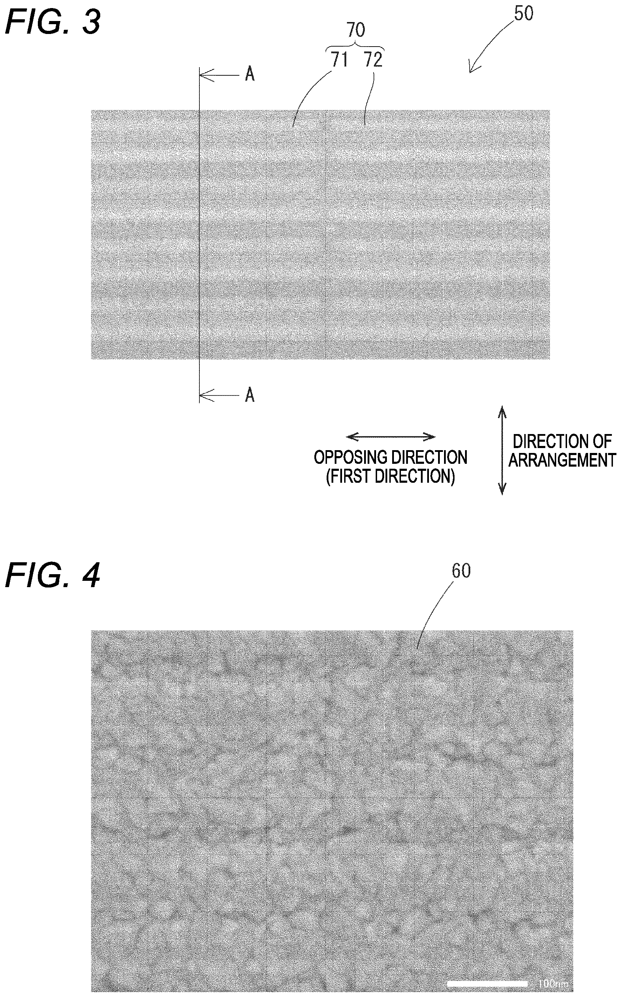

FIG. 3 shows, as an example, the electrode portion 50 including a plurality of electrode pairs 70. As shown in FIG. 3, in each electrode pair 70, the first electrode 71 and the second electrode 72 are opposed to each other in a first direction, with a gap formed therebetween. The first direction is a direction which is parallel to an upper surface of the substrate 20 and in which the first electrode 71 and the second electrode 72 extend. The first direction is also a direction in which the first electrode 71 and the second electrode 72 are opposed to each other. The first direction is orthogonal to a direction in which the plurality of first electrodes 71 are arranged and is also orthogonal to a direction in which the plurality of second electrodes 72 are arranged.

The substrate 20 is composed of, for example, a silicon wafer. Although no particular limitation is imposed on the material of the substrate 20, the material is, for example, a semiconductor such as silicon. In the case where a ceramic substrate (e.g., a sapphire substrate, a zirconia substrate, an alumina substrate, or the like) is used for the substrate 20, formation of the insulating layers 31 to 34, which will be described later, can be omitted. No particular limitation is imposed on the planar shape of the substrate 20, and the planar shape of the substrate 20 may be, for example, rectangular or circular. No particular limitation is imposed on the size of the substrate 20, and the substrate 20 may be a rectangular substrate having a length of 0.1 mm to 10 mm measured along each side, or a circular substrate whose area is approximately the same as that of the rectangular substrate. No particular limitation is imposed on the thickness of the substrate 20, and the substrate 20 may have a thickness of, for example, 400 $\mu$m to 500 $\mu$m.

The substrate 20 may have a space portion 21 formed by removing a portion of the substrate 20. The space portion 21 is, for example, a cavity which penetrates the substrate 20 and is open to front and back surfaces thereof, a recess which is open to only one of the front and back surfaces of the substrate 20, or the like. Although no particular limitation is imposed on the shape of the opening 22 of the space portion 21, the sectional shape of the interior of the space portion 21, etc., in general, their shapes are simple shapes such as rectangular shape, circular shape, etc. No particular limitation is imposed on the size of the space portion 21. In the case where the space portion 21 is a cavity, the space portion 21 is preferably formed such that its opening at one of the front and back surfaces of the substrate 20 has a larger opening area as compared with its opening at the other of the front and back surfaces of the substrate 20. Although no particular limitation is imposed on their opening areas, the opening area of the larger opening is preferably set to 0.01 mm$^2$ to 4 mm$^2$, more preferably 0.25 mm$^2$ to 2 mm$^2$. In the case where the space portion 21 is a recess, its opening area may be set to the same range as the cavity.

The electrode portion 50, which will be described later, is insulated from the substrate 20 by the insulating layers 31 to 35 provided on the substrate 20. The insulating layers 31 to 35 may be formed on the entire surface of the substrate 20 or formed on only a portion of the substrate 20. The insulating layers 31, 33, and 35 are stacked on a surface (upper surface) of the substrate 20 on one side. The space portion 21 is provided in the substrate 20. In the case where the substrate 20 has the space portion 21, the insulating layers 31, 33, and 35 cover the opening 22 of the space portion 21 and are supported by the substrate 20. The insulating layers 31, 33, and 35 may be formed to cover the entire opening 22 or may be formed to cover a portion of the opening 22 if the insulating layers 31, 33, and 35 can be supported by the substrate 20. The insulating layers 32 and 34 are stacked on a surface (lower surface) of the substrate 20 on the other side. No particular limitation is imposed on the materials of the insulating layers 31 to 35 so long as they have sufficient insulating properties. Examples of the materials of the insulating layers 31 to 35 include silicon compounds such as SiO$_2$, Si$_3$N$_4$, and SiO$_x$N$_y$ (x and y are arbitrary values). No particular limitation is imposed on the shapes and thicknesses of the insulating layers 31 to 35. A monolayer insulating layer may be provided instead of the insulating layers 31, 33, and 35. By forming a silicon oxide film on the substrate 20, leak current between the electrodes of each electrode pair 70 can be prevented.

A plurality of heating elements 40 are provided in the gas sensor 10. The heating elements 40 are provided on the insulating layer 33 and are covered with the insulating layer 35. Lead portions (not shown) for supplying electric power from an external circuit are connected to the heating elements 40 via contact portions (not shown) for the heating elements (hereinafter referred to as "heating element contact portions"). Contact pads (not shown) are provided on the surfaces of the heating element contact portions. The heating elements 40 generate heat when a voltage is applied thereto. As a result, the temperature of the sensitive membrane 60 increases, whereby the sensitive membrane 60 is activated, and measurement of gas concentration becomes possible. No particular limitation is imposed on the positions where the heating elements 40 are disposed so long as they are located within the insulating layers 31, 33, and 35. In the case where the space portion 21 is formed in the substrate 20, the heating elements 40 are preferably disposed at positions corresponding to the space portion 21. By disposing the heating elements 40 at positions corresponding to the space portion 21, it is possible to suppress dissipation of heat from the heating elements 40 to the substrate 20. Also, since heat can be efficiently transferred to the sensitive membrane 60, the temperature of the sensitive membrane 60 can be controlled more accurately. Notably, the positions corresponding to the space portion 21 refer to a positional relation in which the heating elements 40 at least partially overlap with the space portion 21 in the thickness direction of the substrate 20, and it is more preferred that the entire heating elements 40 overlap with the space portion 21. The heating elements 40 have electrical conductivity, and no particular limitation is imposed on their material. For example, platinum, a platinum alloy, a nickel alloy, a chromium alloy, a nickel-chromium alloy, or the like may be used for the heating elements 40. Of these materials, platinum or a nickel-chromium alloy, which have large temperature coefficients of resistance and whose resistances and temperature coefficients of resistance are less likely to change in repeated use over a long period of time, are preferably used for the heating elements 40.

The electrode portion 50 (one or more electrode pairs 70) is provided on the surface of the insulating layer 35. One end of the first electrode 71 and one end of the second electrode 72 are disposed to face each other with a nano-gap formed therebetween. In the present first embodiment, the nano-gap means a gap of a distance of 100 nm or less between the one end of the first electrode 71 and the one end of the second electrode 72.

Each of the first electrode 71 and the second electrode 72 is formed of a single type of metal or a plurality of types of metals. Examples of the metal used to form the first electrode 71 and the second electrode 72 include gold (Au) and platinum (Pt). The first electrode 71 and the second electrode 72 have, for example, a two-layer structure including a lower layer electrode and an upper layer electrode. For example, the lower layer electrode is formed of titanium (Ti), and the upper layer electrode is formed of platinum (Pt).

Lead portions (not shown) for supplying electric power from the external circuit are connected to the first electrode 71 and the second electrode 72 via contact portions (not shown) for the electrodes (hereinafter referred to as "electrode contact portions") . Contact pads (not shown) are provided on the surfaces of the electrode contact portions.

The sensitive membrane 60 is provided on the upper surface of the first electrode 71, the upper surface of the second electrode 72, and the upper surface of the insulating layer 35. The sensitive membrane 60 is disposed between the first electrode 71 and the second electrode 72 of each electrode pair 70. The sensitive membrane 60 is preferably formed of a metal oxide. The sensitive membrane 60 is preferably formed of an oxide semiconductor. The oxide semiconductor is preferably an n-type semiconductor. Preferred examples of the n-type semiconductor include $SnO_2$, $ZnO$, $In_2O_3$, $Fe_2O_3$, ITO (tin-doped indium), and $WO_3$, whose resistances change easily.

The sensitive membrane 60 is preferably a dense body. The dense body is a body whose porosity is less than 10%. The surface of the sensitive membrane 60 is, for example, the surface of the dense body as shown in FIG. 4. Since the sensitive membrane 60 is densified, electrons easily move in depletion layers, whereby the response speed (rising speed and returning speed) of an electric signal outputted from the output section 90 can be increased.

At least some particles which constitute the sensitive membrane 60 are electrically coupled (bound) with one another. The expression "particles are electrically coupled with one another" is defined as a state in which at least a half of the surface area of a certain particle, excluding end portions, is in contact with the surfaces of other particles, and a path through which electrons having propagated through the certain particle propagate is formed in at least a half of the surface area of the particle. The particles electrically coupled with one another create a state in which electricity flows between the particles (a state in which electrical conduction is enabled). Since the particles which constitute the sensitive membrane 60 are electrically coupled with one another, the contact resistance between the particles can be reduced, whereby movement of electrons in depletion layers becomes easy.

The voltage application section 80 applies a voltage to the electrode portion 50 such that the voltage is applied between the first electrode 71 and the second electrode 72. The voltage application section 80 includes, for example, a voltage control circuit (a constant voltage circuit or the like) which performs an operation of applying a volage to the electrode portion 50 on the basis of electric power supplied from a power source, and a control section (CPU or the like) which controls the voltage control circuit.

The output section 90 outputs an electric signal representing the gas concentration detected by using the electrode portion 50. The output section 90 may include, for example, an amplifier which amplifies the electric signal, etc.

A gas detected by the gas sensor 10 is preferably flammable. The resistance of the gas sensor 10 can be changed by a flammable gas. Preferred examples of the flammable gas include ketones (e.g., acetone), alcohols (e.g., ethanol), carbon monoxide, nitrogen oxides (NO, $N_2O$), aldehydes (e.g., acetaldehyde), sulfide gases (e.g., hydrogen sulfide), and VOC gases (e.g., toluene).

The gas sensor 10 is preferably of a resistance measurement type or of an impedance measurement type.

1-2. Conditions of electrical index in electrode portion

An electrical index in the electrode portion 50 of the gas sensor 10 will be described. A division value obtained by dividing, by an inter-electrode volume, the voltage V applied to the electrode portion 50 by the voltage application section 80 is defined as an electrical index (hereinafter referred to simply as an index) X in the electrode portion 50. The inter-electrode volume B is the product of an inter-electrode distance D and an electrode area S. Namely, the index X is represented by the following Expression (1).

$$X = V/B = V/(D{\times}S) \qquad \text{Expression (1)}$$

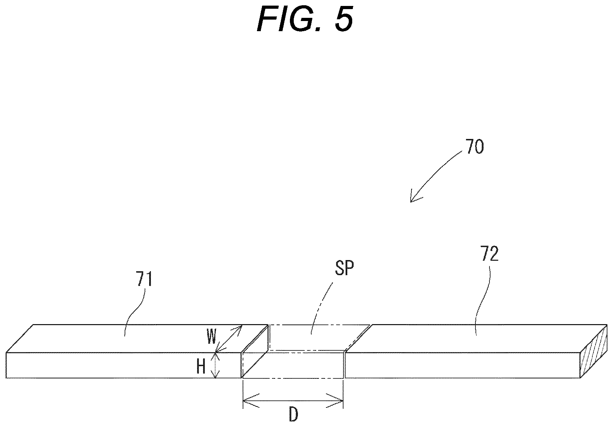

For example, in the case where the electrode pair 70 shown in FIG. 5 is provided ingly, the volume of a space SP between the first electrode 71 and the second electrode 72 corresponds to the inter-electrode volume B.

In the case where the electrode portion 50 is composed of a single electrode pair 70, the inter-electrode distance D is the distance (shortest distance) in the first direction between the first electrode 71 and the second electrode 72 constituting that electrode pair 70. Meanwhile, in the case where the electrode portion 50 is composed of a single electrode pair 70, the inter-electrode distance D is the smallest one of the distances (shortest distances) in the first direction between the first electrodes 71 and the second electrodes 72 in all the electrode pairs 70.

In the case where the electrode portion 50 is composed of a single electrode pair 70, the electrode area S is the electrode area of one electrode (for example, the first electrode 71) of that electrode pair 70. Meanwhile, in the case where the electrode portion 50 is composed of a plurality of electrode pairs 70, the electrode area S is the sum total of the electrode areas of electrodes on one side (for example, the first electrodes 71) of all the electrode pairs 70.

A specific method for obtaining the electrode area in each electrode portion 50 will be described. First, one first electrode 71 is selected (in the case where the electrode portion 50 includes a single electrode pair 70, the first electrode 71 of that electrode pair 70 is selected), and a cut surface is set. For example, the first electrode 71 of the electrode pair 70 for which the above-described inter-electrode distance D was determined is selected. For example, a plane which orthogonally intersects a plate face of the substrate 20 and passes through a position which is offset from a first end (an end portion on the second electrode 72 side) of the selected first electrode 71 toward a second end (an end portion located on the side opposite the second electrode 72) by a distance (preferably, 25 nm) which falls within the range of 10 nm to 100 nm is defined as a cut surface of the electrode portion 50. In the example of FIG. 3, an A-A cross section serves as the cut surface. The area of each first electrode 71 appearing in the cut surface is used as the electrode area. The electrode area S is the total area of the cross sections of all the first electrodes 71 in their cut surfaces obtained by cutting all the first electrodes 71 at a position offset in the first direction from their first ends by a distance in the range of 10 nm to 100 nm.

One example of a method for measuring the electrode area appearing in the cut surface will be described. In the cut surface of the first electrode 71, the width W of the first electrode 71 is defined by the length of the shortest overlap between the cut surface and a straight line which intersects the side surfaces of the first electrode 71 and is parallel to the plate face of the substrate 20. Here, the side surfaces are surfaces located on the left and right sides of the cut surface and each including a portion having an inclination of 45° or less in relation to a straight line orthogonally intersecting the plate face of the substrate 20 (also referred to as an orthogonal line) and a portion located below that portion. In the case where the left and right sides of the cut surface are curved, each side surface is a portion where a tangent line to a curved portion has an inclination of 45° or less in relation to the orthogonal line. Also, in the cut surface of the first electrode 71, the height H of the first electrode 71 is defined by the length of the shortest overlap between the cut surface and an orthogonal line intersecting the upper surface of the first electrode 71. The product of the width W and the height H of the first electrode 71 is defined as the electrode area appearing in the cut surface of the first electrode 71. Here, the upper surface refers to a surface portion which is located at a center of the cut surface in the left and right direction and is other than the side surfaces.

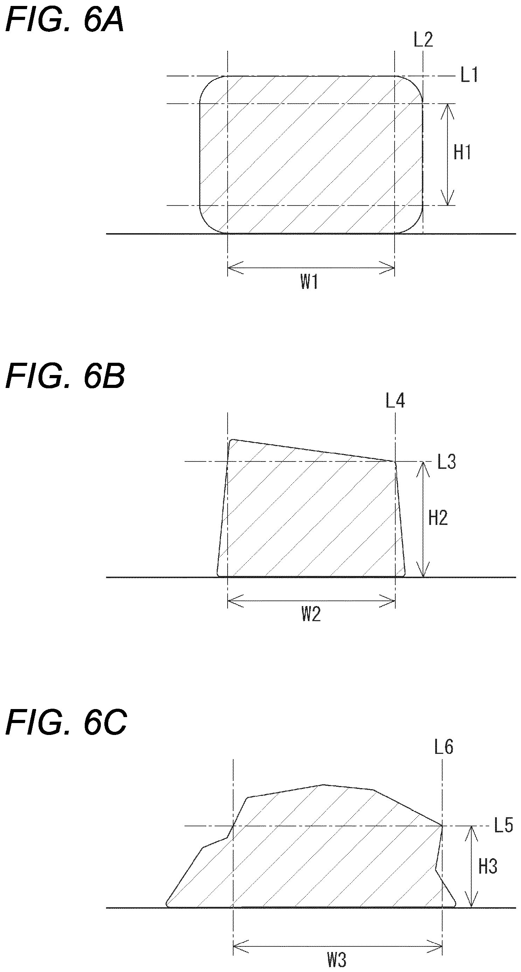

For example, in the cut surface of the first electrode 71 as shown in FIG. 6 (A), a straight line L1 parallel to the plate face of the substrate 20 overlaps with the cut surface with a shortest overlap, and its overlapping length W1 is the width. Also, a straight line L2 orthogonally intersecting the plate face of the substrate 20 overlaps with the cut surface with a shortest overlap, and its overlapping length H1 is the height. Similarly, in the cut surface of the first electrode 71 as shown in FIG. 6 (B), a straight line L3 parallel to the plate face of the substrate 20 overlaps with the cut surface with a shortest overlap, and its overlapping length W2 is the width. Also, a straight line L4 orthogonally intersecting the plate face of the substrate 20 overlaps with the cut surface with a shortest overlap, and its overlapping length H2 is the height. Similarly, in the cut surface of the first electrode 71 as shown in FIG. 6 (C), a straight line L5 parallel to the plate face of the substrate 20 overlaps with the cut surface with a shortest overlap, and its overlapping length W3 is the width. Also, a straight line L6 orthogonally intersecting the plate face of the substrate 20 overlaps with the cut surface with a shortest overlap, and its overlapping length H3 is the height.

Notably, the electrode area S may be obtained by selecting a predetermined number of first electrodes 71 (for example, 100 first electrodes 71) from the plurality of first electrodes 71, calculating the average of cross sectional areas of the selected first electrodes 71, and multiplying the obtained average by the number of the first electrodes 71 contained in the electrode portion 50.

The index in the electrode portion 50 (the index X represented by Expression (1)) is greater than $4.2 \times 10^{16}$ V/m$^3$ and not greater than $2.0 \times 10^{25}$ V/m$^3$, preferably not less than $6.0 \times 10^{19}$ V/m$^3$ and not greater than $2.0 \times 10^{25}$ V/m$^3$, more preferably not less than $6.0 \times 10^{20}$ V/m$^3$ and not greater than $2.0 \times 10^{25}$ V/m$^3$. By virtue of such a configuration, the response speed (rising and returning speeds) of the electric signal outputted by the output section 90 can be increased.

1-3. Conditions of the electrode area

The above-described electrode area S is not less than $2.5 \times 10^{-15}$ m$^2$ and not greater than $2.1 \times 10^{-11}$ m$^2$, preferably not less than $2.1 \times 10^{-14}$ m$^2$ and not greater than $2.1 \times 10^{-12}$ m$^2$. Notably, in the case where the electrode portion 50 is composed of a single electrode pair 70, the electrode area S is the electrode area of the first electrode 71 of that electrode pair 70, and, in the case where the electrode portion 50 is composed of a plurality of electrode pairs 70, the electrode area S is the sum total of the electrode areas of the first electrodes 71 of all the electrode pairs 70. By virtue of such a configuration, the response speed (rising and returning speeds) of the electric signal outputted by the output section 90 can be increased.

1-4. Conditions of the inter-electrode distance

The shortest inter-electrode distance among all the electrode pairs 70 in the electrode portion 50 is preferably not less than 5 nm and not greater than 100 nm. By virtue of such a configuration, a tunnel current effect is obtained between the electrodes, whereby the response speed (rising and returning speeds) of the electric signal outputted by the output section 90 can be further increased.

1-5. Conditions of the total of the widths of the first electrodes

The total of the widths W of the first electrodes 71 of all the electrode pairs 70 in the electrode portion 50 (in the case where the electrode portion 50 includes a single electrode pair 70, the width W of the single first electrode 71) is preferably equal to or greater than 20 nm. By virtue of such a configuration, it is possible to reduce the resistance to electron movement, thereby increasing the response speed (rising and returning speeds) of the outputted electric signal.

1-6. Method for manufacturing the gas sensor

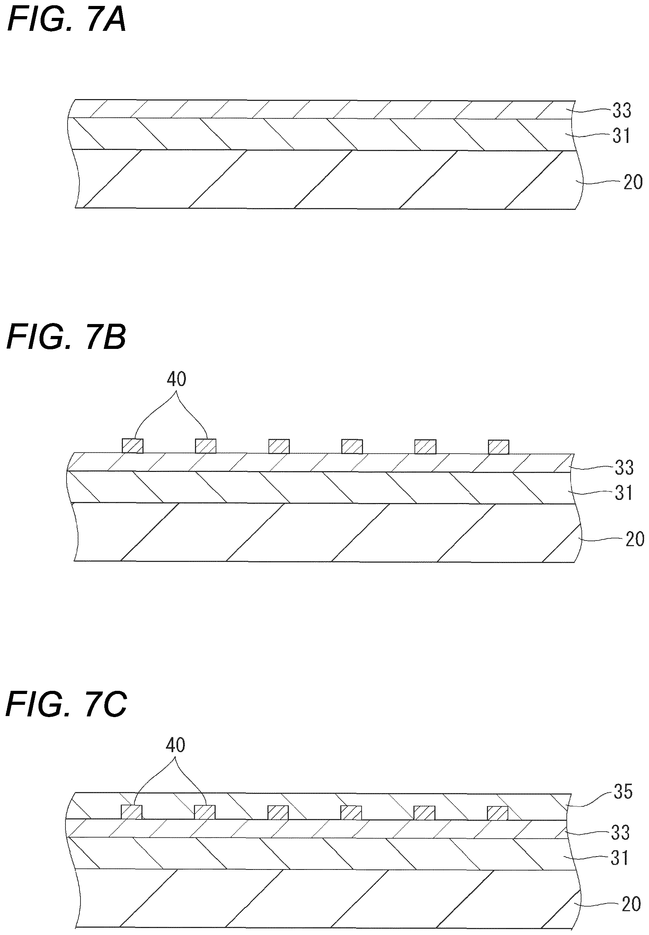

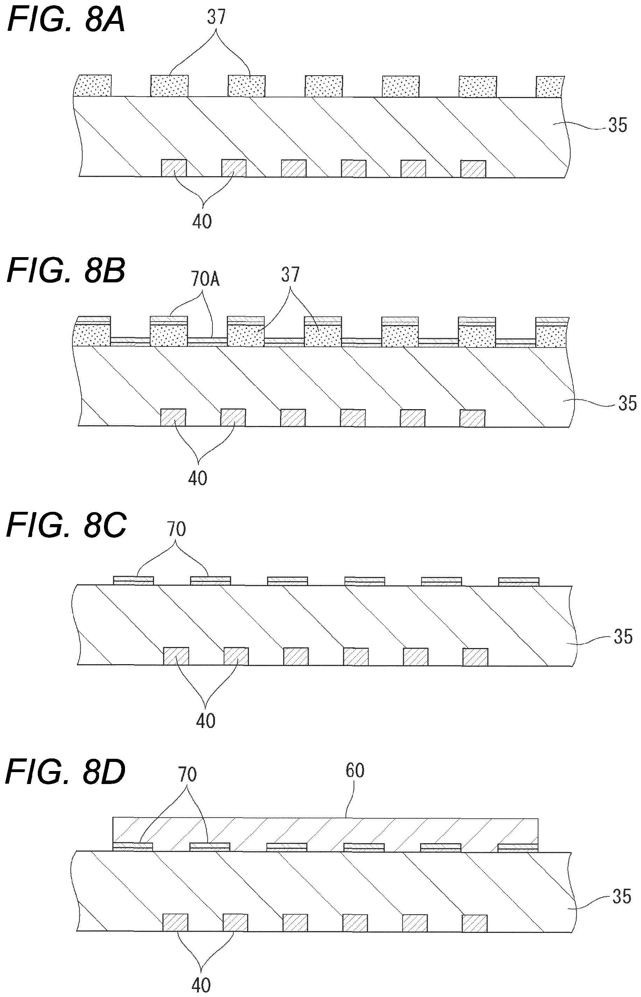

A method for manufacturing the gas sensor 10 in which the present disclosure is embodied will be described with reference to FIGS. 7 and 8.

The method for manufacturing the gas sensor 10 includes a step of forming the electrode portion 50 on the substrate 20 by a lift-off method and a step of forming the sensitive membrane 60 in a gap portion of the electrode portion 50.

First, insulating layers 31, 33, and 35 and heating elements 40 are formed on a cleaned substrate 20 (silicon wafer). The cleaned substrate 20 is placed in a heat treatment furnace, and a silicon oxide film (see FIG. 7(A)) which becomes the insulating layer 31 (first insulating layer) is formed over the entire surface of the substrate 20 by means of thermal oxidation treatment. Notably, in FIGS. 7(A) to (C) and FIGS. 8(A) to (D), only the structure on the upper surface side of the substrate 20 is shown, and description regarding formation of the insulating layers 32 and 34 is omitted. Subsequently, as shown in FIG. 7(A), a silicon nitride film is formed on the insulating layer 31 by means of plasma CVD performed by using, for example, $SiH_4$ and $NH_3$ as source gases. The formed silicon nitride film serves as the insulating layer 33 (lower insulating layer). Subsequently, as shown in FIG. 7(B), the heating elements 40 are formed on the surface of the insulating layer 33 by, for example, a sputtering method. For example, each heating element 40 is composed of a Ti layer and a Pt layer located thereabove. Next, patterning of resist is performed by means of photolithography, and the pattern of the heating elements 40 is formed by means of etching treatment.

No particular limitation is imposed on the method for forming the heating elements 40. For example, a component which becomes the heating element 40 is caused to adhere to and deposit on the surface of the insulating layer 33, and then, unnecessary portions are removed by various types of etching methods, which are similar to the method described as an example in the description of the method for forming the space portion 21. Subsequently, as shown in FIG. 7(C), the insulating layer 35, which is formed of, for example, a silicon nitride film, is formed on the surface of the insulating layer 33 (the insulating layer 35 serves as an upper insulating layer). In this manner, the insulating layers 33 and 35 (second insulating layer) and the heating elements 40 disposed in the insulating layers 33 and 35 (second insulating layer) are formed. Notably, components which become the insulating layers 31 to 35 may be caused to adhere to and deposit on the surface of the substrate 20, thereby forming these layers, or these layers may be formed by bonding previously formed insulating layers to the surface of the substrate.

Subsequently, as shown in FIG. 8(A), a photoresist composition is applied onto the insulating layer 35 by means of spin coating and is dried, whereby a resist film 37 is formed. Notably, in FIGS. 8(A) to (D), the structure of a portion below the insulating layer 35 and the heating elements 40 is not shown. The resist film 37 is formed to a thickness of, for example, 20 nm to 40 nm. The resist film 37 is formed by using a photoresist composition for electron beam exposure. A mask pattern is formed by exposing the resist film 37 by means of electron beam lithography and performing development. The mask pattern is formed such that the gap length of each electrode pair 70 becomes, for example, 20 nm.

Subsequently, as shown in FIG. 8(B), a metal film 70A is formed to cover almost the entire surfaces of the insulating layer 35 and the resist film 37. The metal film 70A is formed by using gold (Au) or platinum (Pt). The metal film 70A preferably has a thickness of 5 nm to 20 nm and is formed to have a thickness of, for example, 15 nm. Such metal film 70A is formed by means of, for example, electron beam deposition. The mask pattern is peeled off, and, simultaneously, portions of the metal film 70A overlapping that portion are removed. As a result, as shown in FIG. 8(C), an electrode portion 50 (a plurality of electrode pairs 70) in which the metal film 70A is stacked is formed. For example, the inter-electrode distance (nano-gap length) in each electrode pair 70 is, for example, 20 nm. Each electrode pair 70 is formed such that the width of the first electrode 71 becomes, for example, 15 nm. Instead of using the above-described electron beam lithography, formation of the electrode pairs 70 may be performed by using nanoimprint lithography in which a mold (metal die) serving as an original plate is pressed against a resist film, thereby transferring a pattern to the resist film. Notably, after formation of the electrodes, annealing (for example, heat treatment at 360°C) may be performed in vacuum.

In the case where, as shown in FIG. 1, the space portion 21 is provided in the substrate 20, the space portion 21 can be formed by, for example, removing a portion of the substrate 20 by means of etching. No particular limitation is imposed on the etching method, and wet etching or dry etching may be used. Also, the etching may be anisotropic etching or isotropic etching. Notably, in the case where a cavity is formed in the substrate 20, a wet etching method using an anisotropic etchant is preferred.

Subsequently, as shown in FIG. 8(D), a sensitive membrane 60 is formed on the upper surfaces of the first electrodes 71, the upper surfaces of the second electrodes 72, and the upper surface of the insulating layer 35. Preferably, the sensitive membrane 60 is formed by a physical vapor deposition method (PVD method) or a chemical vapor deposition method (CVD method). An example of the physical vapor deposition method (PVD method) is a sputtering method. The sensitive membrane 60 is formed by an RF sputtering device in which, for example, a tin oxide ($SnO_2$) target for sputtering is used.

Heat treatment may be performed for the sensitive membrane 60 after being formed. As a result, particles contained in the sensitive membrane 60 are bonded together, whereby the sensitive membrane 60 becomes dense. The heat treatment is performed by using the heater (heating element 40) provided in the gas sensor, and the temperature is changed by supplying electricity to the heater. As a result, the particles constituting the sensitive membrane 60 are easily bonded together , whereby the contact resistance between the particles can be reduced, and movement of electrons in depletion layers becomes easy.

1-7. Effects of the first embodiment

The following description relates to an example of the effect of the present configuration.

In the gas sensor 10 of the first embodiment, when the product of the inter-electrode distance D in the first direction between the first electrode 71 and the second electrode 72 and the electrode area S of the first electrode 71 in a cut surface orthogonal to the first direction is defined as an inter-electrode volume B, the division value (index X) obtained by dividing, by the inter-electrode volume B, the voltage applied to the electrode portion 50 by the voltage application section 80 is greater than $4.2 \times 10^{16}$ V/m³ and not greater than $2.0 \times 10^{25}$ V/m³. The electrode area S is equal to or greater than $2.5 \times 10^{-15}$ m². In this configuration, since the division value obtained by dividing the voltage applied to the electrode portion 50 by the inter-electrode volume B is greater than $4.2 \times 10^{16}$ V/m³ and not greater than $2.0 \times 10^{25}$ V/m³, and the electrode area S is equal to or greater than $2.5 \times 10^{-15}$ m², the response speed (rising and returning speeds) of an outputted electric signal can be increased.

Since the gas sensor 10 of the first embodiment includes 4 or more electrode pairs 70 and is configured such the electrode area S, which is the sum total of the respective electrode areas, becomes equal to or greater than $2.5 \times 10^{-15}$ m², the electrode area S becomes greater than that of the conventional technique. Thus, the response speed (rising and returning speeds) of the outputted electric signal can be increased.

In the gas sensor 10 of the first embodiment, since the division value is not less than $6.0 \times 10^{19}$ V/m³ and not greater than $2.0 \times 10^{25}$ V/m³, the response speed (rising and returning speeds) of the outputted electric signal can be further increased.

In the gas sensor 10 of the first embodiment, the inter-electrode distance which is the smallest among all the electrode pairs 70 in the electrode portion 50 is 5 nm to 100 nm. By virtue of this, a tunnel current effect is obtained between the electrodes, whereby the response speed (rising and returning speeds) of the outputted electric signal can be further increased.

In the gas sensor 10 of the first embodiment, the total of the widths of the first electrodes 71 of all the electrode pairs 70 in the electrode portion 50 is equal to or greater than 20 nm. By virtue of this, the resistance between the first electrodes and the second electrodes can be reduced, whereby the response speed (rising and returning speeds) of the outputted electric signal can be further increased.

In the gas sensor 10 of the first embodiment, the sensitive membrane 60 is a dense body. Therefore, movement of electrons in depletion layers becomes easy, and the response speed (rising and returning speeds) of the outputted electric signal can be increased.

In the gas sensor 10 of the first embodiment, at least some particles which constitute the sensitive membrane 60 are electrically coupled with one another. Since the particles which constitute the sensitive membrane 60 are coupled with one another so as to enable electrical conduction, the contact resistance between the particles can be reduced, and, thus, movement of electrons in depletion layers becomes easy.

In the method for manufacturing the gas sensor 10 of the first embodiment, the sensitive membrane 60 is formed by means of physical vapor deposition or chemical vapor deposition. Therefore, the sensitive membrane 60 can be homogeneously formed, and, thus, stable output of an electric signal becomes possible.

In the method for manufacturing the gas sensor 10 of the first embodiment, heat treatment is performed for the sensitive membrane 60 after being formed. As a result, the particles which constitute the sensitive membrane 60 are easily coupled with one another, and the contact resistance between the particles can be reduced. Therefore, movement of electrons in depletion layers becomes easy.

Examples

The present disclosure will now be described further specifically by means of examples.

1. Manufacture of gas sensors

Gas sensors of Experimental Examples 1 to 6 and Comparative Examples 1 and 2 shown in Table 1 were manufactured by the steps which will be described below. Experimental Examples 1 to 6 correspond to examples.

A silicon substrate (hereinafter also referred to simply as a substrate) of 3 mm × 5 mm was used for the gas sensors of Experimental Examples 1 to 6 and Comparative Examples 1 and 2. An insulating layer was formed on the front and back surfaces of the substrate. The insulating layer is composed of a first insulating layer formed of silicon oxide ($SiO_2$) and a second insulating layer stacked on the surface of the first insulating layer and formed of silicon nitride ($Si_3N_4$).

[Table 1]

Table 1

| | Thickness of sensitive membrane (nm) | Application voltage (V) | Inter-electrode distance (shortest) | Sum total of electrode areas (m²) | Number of electrode pairs | Index X (V/m³) | Aceton sensitivity @100 ppm | Rising responsiveness (T90) | Returning responsiveness (T90) | Evaluation |
|---|---|---|---|---|---|---|---|---|---|---|
| Experimental Example 1 | 20 | 2.5 | 20 nm | $2.1\times10^{-13}$ | 580 | $6.0\times10^{20}$ | 0.17 | 4 seconds | 30 seconds | A |
| Experimental Example 2 | 20 | 0.25 | 20 nm | $2.1\times10^{-13}$ | 580 | $6.0\times10^{19}$ | 0.40 | 5 seconds | 150 seconds | A |
| Experimental Example 3 | 20 | 0.025 | 20 nm | $2.1\times10^{-13}$ | 580 | $6.0\times10^{18}$ | 0.53 | 5 seconds | 300 seconds | B |
| Experimental Example 4 | 200 | 2.5 | 20 nm | $2.1\times10^{-13}$ | 580 | $6.0\times10^{20}$ | 0.17 | 4 seconds | 15 seconds | A |
| Experimental Example 5 | 200 | 0.25 | 20 nm | $2.1\times10^{-13}$ | 580 | $6.0\times10^{19}$ | 0.40 | 5 seconds | 150 seconds | A |
| Experimental Example 6 | 200 | 0.025 | 20 nm | $2.1\times10^{-13}$ | 580 | $6.0\times10^{18}$ | 0.48 | 4 seconds | 300 seconds | B |
| Comparative Example 1 | 20 | 2.5 | 20 μm | $3\times10^{-12}$ | 1 | $4.2\times10^{16}$ | 0.58 | 5 seconds | Did not return for 600 seconds or more | C |
| Comparative Example 2 | 200 | 2.5 | 20 μm | $3\times10^{-12}$ | 1 | $4.2\times10^{16}$ | 0.87 | 5 seconds | Did not return for 600 seconds or more | C |

A space portion was formed in the substrate in such a manner that the space portion is open to the surface on the side where the insulating layer was formed. The area of the opening of the space portion was 1 mm$^2$. Heating elements are formed in the insulating layer at positions corresponding to the space portion. Heating element lead portions (not shown) for supplying electric power are connected to the heating elements. The heating element lead portions have contact portions for connection to an external circuit. The heating elements and the heating element lead portions have a two-layer structure composed of a Pt layer and a Ti layer.

One pair or plural pairs of electrodes are formed on the surface of the insulating layer at positions corresponding to the heating elements. A sensitive membrane is formed on the surface of the insulating layer at a position corresponding to the heating elements. The sensitive membrane is formed on the surface of the insulating layer in such a manner that the sensitive membrane comes into contact with the upper surfaces of the electrodes.
Electrode lead portions are connected to the electrodes. The electrode lead portions have electrode contact portions for connection to the external circuit.

Each of the electrodes of Experimental Examples 1 to 6 has a lower layer electrode formed on the surface of the insulating layer and an upper layer electrode formed on the upper surface of the lower layer electrode. The lower layer electrode is formed of Ti. The upper layer electrode is formed of Pt. The thickness of the lower layer electrode is 3 nm. The thickness of the upper layer electrode is 10 nm.

Each of the electrodes of Comparative Examples 1 and 2 has a lower layer electrode formed on the surface of the insulating layer and an upper layer electrode formed on the upper surface of the lower layer electrode. The lower layer electrode is formed of Ti. The upper layer electrode is formed of Pt. The thickness of the lower layer electrode is 20 nm. The thickness of the upper layer electrode is 40 nm.

(1) Cleaning of substrate

A silicon substrate having a thickness of 400 $\mu$m was immersed in a cleaning solution and cleaning was performed.

(2) Formation of insulating layer

The cleaned silicon substrate was placed in a heat treatment furnace, and a silicon oxide film having a thickness of 100 nm, which would become an insulating layer (first insulating layer), was formed on the entire surface of the substrate by means of thermal oxidation treatment.

(3) Formation of insulating layer and heating elements (including heating element lead portions)

A silicon nitride film was formed on the first insulating layer of the substrate by means of plasma CVD in which $SiH_4$ and $NH_3$ were used as source gases. A silicon nitride film having a thickness of 400 nm was formed on the surface (upper surface) of the substrate on one side and used as an insulating layer (lower insulating layer). A silicon nitride film having a thickness of 200 nm was formed on the surface (lower surface) of the substrate on the other side and used as an insulating layer (lower insulating layer). After that, heating elements were formed on the surface of the second insulating layer by using a DC sputtering device. Specifically, a Ti layer having a thickness of 25 nm was formed on the surface of the second insulating layer, and a Pt layer having a thickness of 250 nm was further formed on the surface of this Ti layer, whereby the heating elements were formed. Next, patterning of resist was performed by means of photolithography, and the pattern of the heating elements was formed by means of etching. After that, a silicon nitride film having a thickness of 400 nm, which would become the upper insulating layer of the second insulating layer, was formed in the same manner as in the case of the lower insulating layer. In this manner, the second insulating layer and the heating elements disposed in the second insulating layer were formed.

(4) Formation of heating element contact portions

The insulating layers were etched by means of dry etching so as to remove portions corresponding to the heating element contact portions, thereby exposing portions (portions of the heating elements) which would become the heating element contact portions. After that, by using a DC sputtering device, a Ti layer having a thickness of 20 nm was formed, and a Pt layer having a thickness of 40 nm was then formed. After sputtering, patterning of resist was performed by means of photolithography, whereby the heating element contact portions were formed.

(5) Formation of contact pads

By using a DC sputtering device, a Cr layer having a thickness of 50 nm was formed on the substrate, and an Au layer having a thickness of 1 μm was formed on its surface. After that, patterning of resist was performed by means of photolithography, and, by means of etching, respective contact pads were formed on the surfaces of the electrode contact portions and the heating element contact portions.

(6) Formation of space portion

The substrate was immersed into a TMAH (tetramethylammonium hydroxide) solution, and anisotropic etching of silicon was performed so as to form a space portion at a position corresponding to the heating elements. The space portion was formed such that an opening was formed in the surface on which the insulating layers were formed.

(7) Formation of sensitive membrane

Tin oxide ($SnO_2$) was used as nanoparticles of a metal oxide provided at nanogap portions of the nanogap electrodes. A membrane was formed by using an RF sputtering device in which a tin oxide target for sputtering was used. The substrate was heated to 280°C, and tin oxide was sputtered, while sputtering to regions other than a region where a sensitive membrane was to be formed was prevented by a mask, whereby a sensitive membrane was formed. At that time, a membrane having a thickness of 20 nm or 200 nm was formed. FIG. 4 shows an SEM (scanning electron microscope) image obtained by observing the formed sensitive membrane. From FIG. 4, it is understood that the sensitive membrane is a dense film in which particles are bonded together and whose porosity is 10% or less. The particles of the sensitive membrane are electrically coupled (bonded) with one another.

Notably, a bonding treatment (heat treatment) may be further performed so as to produce a more dense sensitive membrane. The heat treatment at that time was performed by using the heater (the heating elements 40) provided in the gas sensor. Specifically, by applying electricity to the heater, the temperature was elevated, and, in clean air, the gas sensor was heated at 300°C to 600°C for 10 minutes to 5 hours. Notably, it is sufficient that oxygen coexists so as to prevent reduction of tin oxide, and, therefore, the atmosphere may be a simulated gas produced from a gas cylinder or the like.

2. Gas sensor evaluation method

Table 1 shows the results of evaluation performed for the manufactured gas sensors by using a gas sensor evaluation device 100 shown in FIG. 9. The gas sensor evaluation device 100 includes a sensor evaluation section 101 and mass flow controllers 102 and 103. For example, an electric signal outputted from the output section 90 of each gas sensor 10 is inputted to the sensor evaluation section 101, and evaluation is performed. In Experimental Examples 1 to 6, gas sensors having 580 electrode pairs were used so as to prevent the electric field strength from becoming excessively high. In the gas sensors of Experimental Example 1 to 6, the inter-electrode distance (shortest inter-electrode distance) was 20 nm, the height of the first electrode was 13 nm, and the width of the first electrode was 27.5 nm. As will be described later, the height and the width of the first electrode described here were averaged values (averaged first electrode height and width) of 100 electrode pairs randomly selected from the 580 electrode pairs. The height of the upper layer electrode (Pt layer) was 10 nm, and the height of the lower layer electrode (Ti layer) was 3 nm. As will be described later, the height of the Pt layer and the height of the Ti layer were averaged values (averaged Pt layer height and Ti layer height) of 100 electrode pairs randomly selected from the 580 electrode pairs. In the gas sensors of Comparative Examples 1 and 2, the inter-electrode distance was 20 μm, the height of the first electrode was 60 nm, and the width of the first electrode was 50 μm. The height of the upper layer electrode (Pt layer) was 40 nm, and the height of the lower layer electrode (Ti layer) was 20 nm.

Notably, the inter-electrode distance (shortest inter-electrode distance) was measured through planar observation performed, by using an SEM (scanning electron microscope), in a state in which the electrode portion was formed. In Experimental Examples 1 to 6, the width and the height of the first electrode in its cut surface were averaged values (averaged first electrode height and width) of 100 electrode pairs randomly selected from the 580 electrode pairs. The cut surface was mirror-polished and was observed under an SEM (scanning electron microscope) for measurement.

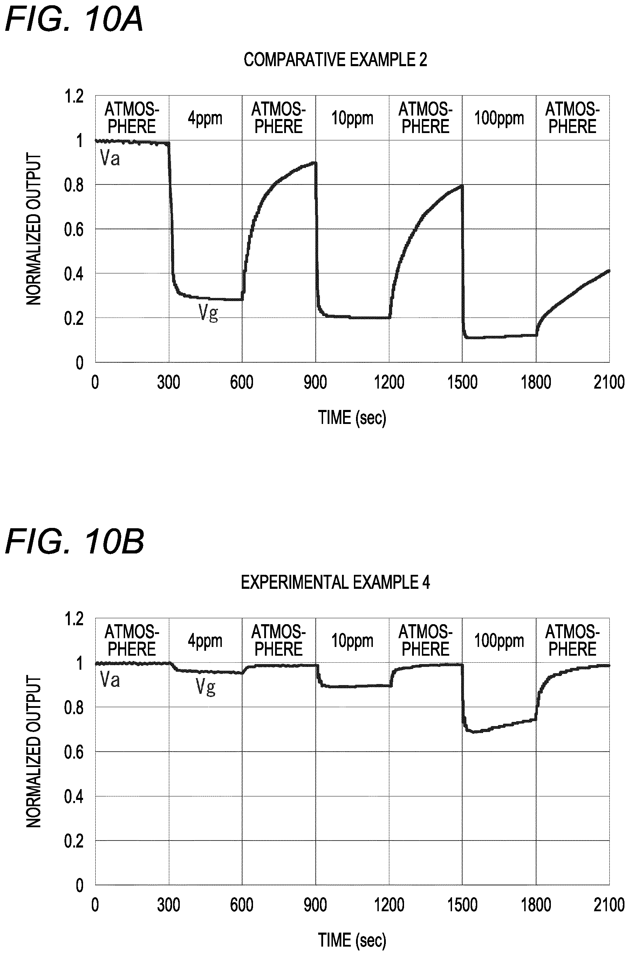

Acetone was used as a gas to be detected. Measurement was performed by using purified air as a base gas and using acetone supplied from a 2000 ppm/$N_2$ balance cylinder (product of TAKACHIHO CHEMICAL INDUSTRIAL CO., LTD.) Acetone was mixed with purified air by using the mass flow controllers 102 and 103 such that the concentration of acetone became a predetermined concentration (here 4, 10, or 100 ppm). The measurement flow rate was 200 sccm. The measurement atmosphere was controlled by using the heater (heating elements) disposed in each gas sensor. Specifically, electric power supplied to the heater was controlled such that the temperature of the gas sensor became 250°C. The heater temperature was controlled by using the previously calculated correlation between the heater temperature and the heater resistance; i.e., by supplying electric power to the heater in accordance with the heater resistance. FIG. 10 shows the results of measurement of responsiveness when acetone was added. The base resistance varies among elements. The base resistance and sensitivity were normalized such that the value of the base resistance became 1. In Experimental Examples 1 and 4 and Comparative Examples 1 and 2, measurement was performed by applying a voltage of 2.5 V between the electrodes.

FIG. 10(A) is an explanatory graph showing changes in output with time in the gas sensor of Comparative Example 2, and FIG. 10(B) is an explanatory graph showing changes in output with time in the gas sensor of Experimental Example 4. These graphs show that, as compared with the gas sensor of Comparative Example 2, the gas sensor of Experimental Example 4 has higher responsiveness. Namely, when acetone was added, the sensor resistance of the gas sensor of Experimental Example 4 decreased with good responsiveness, and, when the gas supplied to the gas sensor of Experimental Example 4 became the atmosphere, the gas sensor of Experimental Example 4 exhibited good responsiveness. The atmosphere, acetone (4 ppm), the atmosphere, acetone (10 ppm), the atmosphere, acetone (100 ppm) were supplied to the gas sensor in this order. The output voltage of the gas sensor changes in accordance with the concentration. A change in the sensor resistance can be converted to voltage by using a measurement circuit, and the measurement circuit can output a voltage corresponding to the resistance. In FIG. 10, Va represents the normalized base resistance, and Vg represents a change from the base resistance when the gas sensor exhibited sensitivity.

As to rising time and returning time, T90 represents a time needed to reach 90% of an initial value (a value at the time when sensitivity saturates), which is considered as 100%. Similarly, T50 represents a time needed to reach 50% of the initial value (a value at the time when sensitivity saturates), which is considered as 100%. In Experimental Example 4, the rising responsiveness (T90 response speed) at the time of exhibition of sensitivity was 5 seconds or less, and the output returned to the original value within 15 seconds in a recovery period. A conceivable reason of this is that the inter-electrode electric field strength is high.

In Experimental Example 4, since the voltage applied to the electrode portion is 2.5 V, the electric field strength becomes $6.0 \times 10^{20}$ V/m$^3$. Meanwhile, in the case of Comparative Examples 1 and 2, in which the inter-electrode distance is 20 $\mu$m, the rising responsiveness (T90 response speed) was 30 seconds, which is relatively quick, but the output did not return to a value corresponding the base resistance (Va) within the measurement time; i.e., T50 was 300 seconds or longer, which shows that the recovery properties are very poor . A conceivable reason of this is that, in Comparative Examples 1 and 2, the electric field strength is low unlike Experimental Examples 1 to 6. In Comparative Examples 1 and 2, since the height of the first electrode is 60 nm and the width of the first electrode is 50 $\mu$m, the electric field strength becomes $4.2 \times 10^{16}$ V/m$^3$. The thickness of the $SnO_2$ layer was 200 nm.

In Experimental Examples 2 and 5, the voltage applied to the electrode portion was 0.25 V, and in Experimental Examples 3 and 6, the voltage applied to the electrode portion was 0.025 V. As shown in Table 1, when the applied voltage is lowered to 0.025 V, the returning responsiveness (returning of the output) becomes slightly bad. Namely, this test revealed that it is preferred that the electric field strength is $6.0 \times 10^{18}$ V/m$^3$ or higher.

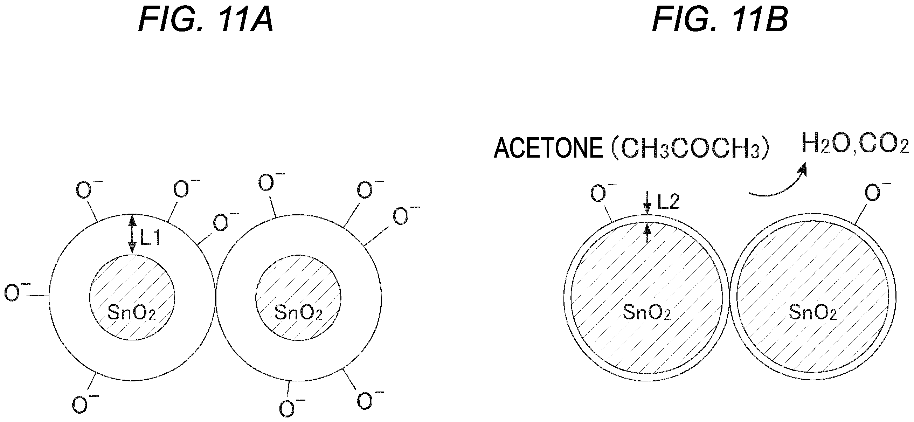

The dependency of current on gas concentration measured in Experimental Examples 1 to 6 shows that the concentration of oxygen vacancies of tin oxide changes with gas. Namely, a conceivable reason of this is that, when the gas sensor is exposed to the gas to be detected and the oxygen partial pressure becomes low, which is shown as a change from FIG. 11(A) to FIG. 11(B), the width of depletion layers of tin oxide decreases from L1 to L2, whereby movement of electrons becomes easy, and, thus, resistance decreases.

<Other embodiments>

The present invention is not limited to the embodiment described by the above description with reference to the drawings, and, for example, embodiments which will be described below are contained in the technical scope of the present invention. Also, various features of the above-described embodiments and the embodiments which will be described below may be combined in any manner so long as a conflict does not occur.

In the above-described first embodiment, as the method for measuring the area of the cut surface of the first electrode 71, a method for measuring the width W and the height H of the cut surface has been shown as an example. However, other methods may be used. For example, the area of the cut surface may be measured by using an analysis software program or the like.

In the above-described first embodiment, the inter-electrode distance of an electrode pair whose inter-electrode distance (shortest distance) is the smallest among the plurality of electrode pairs is employed as the inter-electrode distance D. However, the average of the inter-electrode distances (shortest distances) of all of the plurality of electrode pairs or the average of inter-electrode distances (shortest distances) of a predetermined number of electrode pairs selected from the plurality of electrode pairs may be used.

In the above-described first embodiment, the plane which passes through a certain position which is offset from the first end (an end portion on the second electrode 72 side) of the first electrode 71 by a certain distance which falls within the range of 10 nm to 100 nm is defined as a cut surface, and the conditions of the index X and the electrode area are defined for that cut surface. However, there may be employed a configuration which satisfies the conditions of the above-described index X and the above-described electrode area in any of cut surfaces which pass through positions which are offset from the first end by distances of 10 nm to 100 nm.

**EP 4 517 314 A1**

In the above-described first embodiment, the electrode area S is obtained by calculating the average value of the sectional areas of a predetermined number (for example, 100) of first electrodes 71 among the plurality of first electrodes 71, and multiplying the average value by the number of the first electrodes 71. However, the electrode area S may be obtained by adding together the sectional areas of the all the first electrodes 71, without calculating the average value.

In the above-described first embodiment, gold (Au), platinum (Pt), and titanium (Ti) are shown as examples of the materials of the first electrode 71 and the second electrode 72. However, the materials may be elements which are high in catalytic activity such as palladium(Pd), ruthenium(Ru), rhodium(Rh), and iridium(Ir).

In the above-described first embodiment, the heating elements 40 and the electrode portion 50 are formed on the insulating layer 33 and the insulating layer 35, respectively. However, they may be formed on an insulating film such as a silicon oxide ($SiO_2$) film or an alumina ($Al_2O_3$) film.

Notably, the embodiments disclosed this time should be considered to be illustrative and not to be restrictive in all aspects. The scope of the present invention is not limited to the embodiments disclosed this time, and it is intended that the present invention encompasses all modifications within the range shown by the claims and the range of equivalents of the claims.

REFERENCE SIGNS LIST

10: gas sensor
20: substrate
21: space portion
22: opening
31 to 35: insulating layer
37: resist film
40: heating element
50: electrode portion
60: sensitive membrane
70: electrode pair
70A: metal film
71: first electrode
72: second electrode
80: voltage application section
90: output section
100: gas sensor evaluation device
101: sensor evaluation section
102, 103: mass flow controller

**Claims**

1. A gas sensor **characterized by** comprising:

    an electrode portion which includes one or more electrode pairs each having a first electrode and a second electrode, the first electrode and the second electrode being opposed to each other in a first direction in the electrode pair, with a gap formed therebetween;
    a sensitive membrane disposed between the first electrode and the second electrode in the electrode pair; and
    a voltage application section which applies a voltage to the electrode portion such that the voltage is applied between the first electrode and the second electrode,
    wherein, when the product of an inter-electrode distance in the first direction between the first electrode and the second electrode and an electrode area of the first electrode in a cut surface orthogonal to the first direction is defined as an inter-electrode volume, a division value obtained by dividing, by the inter-electrode volume, the voltage applied to the electrode portion by the voltage application section is greater than $4.2 \times 10^{16}$ V/m$^3$ and not greater than $2.0 \times 10^{25}$ V/m$^3$ and
    wherein the electrode area is equal to or greater than $2.5 \times 10^{-15}$ m$^2$.

2. A gas sensor according to claim 1, wherein

the electrode portion includes 4 or more electrode pairs, and
the electrode area is the total area of cross sections of all the first electrodes in a cut surface obtained by cutting all the first electrodes at a position which is offset in the first direction from their end portions on the second electrode side by an amount of 10 nm to 100 nm.

3. A gas sensor according to claim 1 or 2, wherein the division value is not less than $6.0\times10^{19}$ V/m$^3$ and not greater than $2.0\times10^{25}$ V/m$^3$.

4. A gas sensor according to claim 1 or 2, wherein the inter-electrode distance which is the smallest among all the electrode pairs in the electrode portion is 5 nm to 100 nm.

5. A gas sensor according to claim 1 or 2, wherein the total of widths of the first electrodes of all the electrode pairs in the electrode portion is equal to or greater than 20 nm.

6. A gas sensor according to claim 1 or 2, wherein the sensitive membrane is a dense body.

7. A gas sensor according to claim 6, wherein at least some particles which constitute the sensitive membrane are electrically coupled with one another.

8. A method for manufacturing the gas sensor according to claim 6, the method being **characterized in that** the sensitive membrane is formed by means of physical vapor deposition or chemical vapor deposition.

9. A method for manufacturing the gas sensor according to claim 6, the method being **characterized in that** heat treatment is performed for the sensitive membrane after being formed.

# FIG. 1

## FIG. 2

*FIG. 3*

OPPOSING DIRECTION
(FIRST DIRECTION)

DIRECTION OF
ARRANGEMENT

*FIG. 4*

# FIG. 5

## FIG. 6A

## FIG. 6B

## FIG. 6C

## FIG. 7A

```
33
31
20
```

## FIG. 7B

```
40
33
31
20
```

## FIG. 7C

```
40
35
33
31
20
```

*FIG. 8A*

37

35

40

*FIG. 8B*

70A    37

35

40

*FIG. 8C*

70

35

40

*FIG. 8D*

70    60

35

40

# *FIG. 9*

## FIG. 10A

COMPARATIVE EXAMPLE 2

## FIG. 10B

EXPERIMENTAL EXAMPLE 4

FIG. 11A

FIG. 11B

EP 4 517 314 A1

# INTERNATIONAL SEARCH REPORT

| International application No. |
|---|
| **PCT/JP2023/013804** |

### A. CLASSIFICATION OF SUBJECT MATTER

***G01N 27/12***(2006.01)i
FI:  G01N27/12 B; G01N27/12 M

According to International Patent Classification (IPC) or to both national classification and IPC

### B. FIELDS SEARCHED

Minimum documentation searched (classification system followed by classification symbols)

G01N27/12, G01N27/26-27/49

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | HYODO, T. et al. NO2 sensing properties of macroporous In2O3-based powders fabricated by utilizing ultrasonic spray pyrolysis employing polymethylmethacrylate microspheres as a template. Sensors and Actuators B. 2010, vol. 151, pp. 265-273<br>entire text, in particular, fig. 11 | 1, 5 |
| X | WO 2012/017623 A1 (PANASONIC CORPORATION) 09 February 2012 (2012-02-09)<br>paragraphs [0048]-[0051] | 1, 5 |
| A | JP 2005-315874 A (NEW COSMOS ELECTRIC CORP) 10 November 2005 (2005-11-10)<br>claims, paragraphs [0043], [0062] | 6-7 |
| A | JP 2001-349858 A (FUJI ELECTRIC CO LTD) 21 December 2001 (2001-12-21)<br>claim 1, paragraphs [0011]-[0012], fig. 1-2 | 6-9 |
| A | JP 7-198649 A (LG DENSHI KK) 01 August 1995 (1995-08-01)<br>claims 8-12, paragraphs [0032]-[0051] | 6-9 |
| A | JP 2021-032746 A (TOKYO INST TECH) 01 March 2021 (2021-03-01)<br>entire text, all drawings | 1-9 |

☑ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 June 2023** | **20 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2023/013804** |

**C.     DOCUMENTS CONSIDERED TO BE RELEVANT**

| Category* | Citation of document, with indication, where appropriate, of the relevant passages | Relevant to claim No. |
| --- | --- | --- |
| P, X | JP 2023-042090 A (TORAY INDUSTRIES) 27 March 2023 (2023-03-27)<br>paragraphs [0077]-[0080] | 1, 5 |

Form PCT/ISA/210 (second sheet) (January 2015)

**INTERNATIONAL SEARCH REPORT**
Information on patent family members

International application No.

**PCT/JP2023/013804**

| Patent document cited in search report | | | Publication date (day/month/year) | Patent family member(s) | Publication date (day/month/year) |
|---|---|---|---|---|---|
| WO | 2012/017623 | A1 | 09 February 2012 | US 2012/0184045 A1 paragraphs [0056]-[0059] CN 102687001 A | |
| JP | 2005-315874 | A | 10 November 2005 | (Family: none) | |
| JP | 2001-349858 | A | 21 December 2001 | (Family: none) | |
| JP | 7-198649 | A | 01 August 1995 | US 5605612 A column 6, line 66 to column 9, line 26 KR 10-1995-0014873 A CN 1113321 A | |
| JP | 2021-032746 | A | 01 March 2021 | (Family: none) | |
| JP | 2023-042090 | A | 27 March 2023 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021032746 A